# EUROPÄISCHE PATENTANMELDUNG

(11) **EP 0 967 223 A2**
(43) Veröffentlichungstag der Anmeldung: **29.12.1999**
(21) Anmeldenummer: 99113825.6
(22) Anmeldetag: 02.08.1993
(51) Int. Cl.: C07K 14/18, A61K 39/29, G01N 33/576

(54) **Hepatitis C Virus Peptidantigene und Verfahren zur Bestimung von Hepatitis C Virus (HCV)**

(30) Priorität: 07.08.1992 DE 4226093; 05.12.1992 DE 4240980
(62) Teilanmeldung aus: 93112337.6
(71) Anmelder: Roche Diagnostics GmbH, 68298 Mannheim (DE)
(72) Erfinder: Seidel, Christoph, Dr., 82362 Weilheim (DE); Wienhues-Thelen, Ursula-Henrike, Dr., 82152 Krailling (DE); Bayer, Hubert, Dr., 69469 Weinheim (DE); Jung, Günther-Gerhard, Prof. Dr., 72076 Tübingen (DE); Ihlenfeldt, Hans Georg, Dr., 82393 Iffeldorf (DE)

(57) **Zusammenfassung**

Es werden neue HCV-Peptidantigene beschrieben. Diese Peptidantigene sind zur Bestimmung von HCV-Antikörpern als Immunogene zur Herstellung von Antikörpern gegen HCV und als Vakzine zur Herstellung von Impfstoffen gegen HCV geeignet.

## Beschreibung

Die Erfindung betrifft neue HCV Peptidantigene, ein Verfahren zur Herstellung dieser Peptidantigene, sowie ein Verfahren zur Bestimmung von HCV unter Verwendung der Peptidantigene.

Das Vorkommen von Virushepatitis in Abwesenheit serologischer Marker von bisher bekannten hepatotropen Agenzien (z.B. Hepatitis-A-Virus, Hepatitis-B-Virus, Hepatitis- - Virus, Cytomegalie-Virus und Epstein-Barr-Virus) wird als Non-A, Non-B-Hepatitis (NANB-Hepatitis) bezeichnet. NANB-Hepatitis wird wiederum unterteilt in parenteral und sporadisch übertragene Non-A, Non-B-Hepatitis und enteral übertragene Non-A, Non-B-Hepatitis. Für die parenteral und sporadisch übertragene NANB-Hepatitis wurde vor kurzem das ursächliche Agenz, das Hepatitis-C-Virus (HCV) isoliert (Choo Q.-L. et. al. Science 244 (1989) 359 - 362 und Kuo, G. et. al. Science 244 (1989) 362 - 364).

HCV ist weltweit eine wichtige Ursache von NANB-Hepatitis und wird durch kontaminiertes Blut oder Blutprodukte, Blutübertragungen oder durch engen persönlichen Kontakt übertragen.

Die Aminosäuresequenz der HCV-Virusproteine ist aus EP-A 0 318 216, EP-A 0 363 025, EPA 388 232 und EP-A 0 396 748 bekannt. Das Genom des HCV hat eine Länge von 10862 nt. Die durch Translation hervorgehenden Proteine besitzen eine Gesamtlänge von ca. 3000 Aminosäuren. Die Proteine lassen sich in Strukturproteine (Hülle- und Kernproteine) und Nicht-Strukturproteine (NS1 - NS5) einteilen.

Die Bestimmung von HCV erfolgt zweckmäßig dadurch, daß durch immunologische Tests Antikörper gegen HCV in Körperflüssigkeiten nachgewiesen werden. Für derartige immunologische Tests werden deshalb Bindepartner für Anti-HCV-Antikörper benötigt.

So ist es bekannt, in immunologischen Tests beispielsweise das nicht-strukturelle C 100-3-HCV-Protein als Bindepartner zu verwenden (Tests von ABBOTT LABORATORIES, USA und ORTHO DIAGNOSTIC SYSTEMS INC., USA; Science 244 (1989) 359 - 364; Van der Poel C. L. et. al. Lancet 337(1991)317; Alter H.J. J. Gastroent. Hepatol. (suppl.) 1990, 78).

Nachteil dieser Tests ist, daß als Antigen ein rekombinantes Protein verwendet wird. Proteine sind wegen ihrer Denaturierungsanfälligkeit und damit verringerter Löslichkeit und Funktion in diagnostischen Tests schwer zu handhaben. Auch die Größe des Meßsignals ist aufgrund der niedrigen Epitopdichte auf einem Protein geringer als bei einem Test, bei dem ein kurzkettiges Peptidantigen als Bindepartner des Antikörpers verwendet wird. Weiter können bei Verwendung von Proteinen oder langkettigen Peptiden als Antigene in einem immunologischen Test vermehrt Kreuzreaktivitäten und unspezifische Bindungen von Antikörpern auftreten. Reaktionen mit Proteinen sind zudem oft diffusionskontrolliert, was den erwünschten kurzen Zeiten für immunologische Tests entgegensteht. Außerdem ist die Herstellung von für die Diagnostik einsetzbarem Protein in ausreichender Menge und Qualität zeitraubend und kostenintensiv. Peptide sind durch Synthese leicht zugänglich und sind definierte Moleküle.

Es ist demzufolge vorteilhaft, in einem immunologischen Test auf Anti-HCV-Antikörper möglichst kurzkettige Peptidantigene, die nur Ausschnitte der gesamten Proteine darstellen, zu verwenden. Ein derartiges immunologisches Verfahren ist von Okamoto (Japan J. Exp. Met. 60 (1990) 223 - 234) beschrieben. Es hat sich jedoch gezeigt, daß das dort beschriebene kurzkettige Peptidantigen (Sequenz 9), welches aus der core-Region stammt, nicht ausreichend sensitiv für HCV ist.

Weitere HCV-Peptidantigene sind in der Deutschen Patentanmeldung P 42 09 215.9 beschrieben.

Aufgabe der vorliegenden Erfindung ist es, Peptidantigene zur Verfügung zu stellen, die spezifisch für Anti-HCV-Antikörper sind und für immunologische Tests auf Anti-HCV-Antikörper geeignet sind.

Diese Aufgabe wird gelöst durch die Peptidantigene der Sequenzen
- 7B6:
   LDGVRLHRFAPPCKPLLR
- 7A5:
   LHQWISSECTTPCSGSWLRDI
- NS5/1:
   SRRFAQALPVWARPD
- 7B12:
   NKVVILGSFDPLVAEEDEREI
- 6F10:
   PSHITAEAAGRRLARG
- 7A1:
   SRGNHVSPTHYVPESDAA
- 8C3:
   LLLLAAGVGIYLLPN
- X:
   GQIVGGVYLLPRCPRLG
oder Peptidantigene, die Teilsequenzen dieser Peptidantigene von mindestens vier, vorzugsweise von mindestens sieben, Aminosäuren Länge darstellen.

Das Epitop in der Sequenz X konnte nicht ohne weiteres gefunden werden. Es war dazu erforderlich, entsprechende Peptide in Lösung mit Antikörpern gegen HCV in Kontakt zu bringen und anschließend die gebildeten Immunkomplexe nachzuweisen. Dies geschah durch Biotinylierung der Peptide und Immobilisierung der Komplexe an einer mit Streptavidin überzogenen Festphase. Die aufgefundenen Peptide sind auch besonders geeignet für Immunoassays, die in-Lösung-Komplexierung verwenden. Das Epitop befindet sich unmittelbar C-terminal hinter dem in WO 93/01210 unter SEQ. ID. NO. 16 beschriebenen Peptid und liegt N-terminal in der core-Region.

Geeignete Teilsequenzen sind in den Sequenzprotokollen dargestellt.

Besonders bevorzugte Teilsequenzen sind:

### aus Sequenz 7A1:

- NS4/3a:
   HVSPTHYVP
- NS4/3b:
   VSPTHYVPE
- NS4/3:
   HVSPTHYVPE

### aus Sequenz NS5/1:

- 7D/2:
   ALPVWARPD
- NS5/1b:
   FAQALPVWA

### aus Sequenz X:

- D2:
   VYLLPR
- D1:
   GVYLLPRR

Besonders bevorzugt werden Teilsequenzen, deren Länge maximal 9 Aminosäuren beträgt, insbesondere die Substanzen NS4/3 und NS5/1b. Ein besonders bevorzugtes Peptidantigen ist NS5/1.

Aus der Sequenz X erkennt SEQ. ID. NO. 26 alle untersuchten Seren, während SEQ. ID. NO. 25 das reaktive Antigen (höchstes Signal) ist.

Die Durchführung eines Anti-HCV-Antikörper-Nachweis erfolgt nach den dem Fachmann geläufigen Methoden. Ein weiterer Gegenstand der Erfindung ist deshalb ein Verfahren zur Bestimmung von HCV-Antikörpern, welches dadurch gekennzeichnet ist, daß die Probe mit mindestens einem Peptidantigen aus der Gruppe der Sequenzen SEQ ID NO 1 - 11, 20 und 22-28 oder Peptid-antigenen, die Teilsequenzen dieser Peptidantigene von mindestens vier, vorzugsweise von mindestens 7, Aminosäuren Länge darstellen, inkubiert und unter Bedingungen, die die Bildung eines Antikörper-Antigenkomplexes ermöglichen, die Menge der an das Peptidantigen gebundenen Anti-HCV-Antikörper bestimmt wird.

Dabei werden die erfindungsgemäßen Peptidantigene vorzugsweise in einem Konzentrationsbereich von 1 - 1000 ng/ml, besonders besonders bevorzugt von 20 - 250 ng/ml eingesetzt.

Erfindungsgemäß wird eine Kombination von mindestens zwei der erfindungsgemäßen Peptidantigene oder Teilsequenzen davon bevorzugt verwendet. Besonders bevorzugt ist es, mindestens ein Peptidantigen der Sequenzen SEQ ID NO: 1 - 11, 20 und 22-28 oder Teilsequenzen davon mit mindestens einem Peptidantigen aus der Gruppe der Sequenzen SEQ ID NO: 12 - 19 oder Teilsequenzen davon zu kombinieren.

Die Sequenzen SEQ ID NO: 12 - 19 und ihre Herstellung sind in der deutschen Patentanmeldung P 42 09 215.9, deren Inhalt Gegenstand der Offenbarung der vorliegenden Patentanmeldung ist, beschrieben.

Die Kombination der Antigene kann beispielsweise dadurch erfolgen, daß mehrere einzelne Peptidantigene verwendet werden oder daß Peptidantigene kovalent, zweckmäßig über eine Aminosäurenbrücke, die sich von natürlicherweise in HCV-Proteinen vorkommenden Aminosäuren-Sequenzfolgen unterscheidet oder einen Peptid-Linker, aneinander gebunden sind.

Besonders bevorzugt sind Kombinationen der Antigene:
Sequenz 4a, 6e, 6b, 2e, 2g, NS4/3, NS5/1 (Kombination 1)
Sequenz 4a, 6e, 6b, 2e, 2g, NS4/3 (Kombination 2)
Sequenz 4a, 2g, 2e, 6c, NS4/3, NS5/1 (Kombination 3)
Sequenz 4a, 6e, 6b, 2e, 2g, NS4/3a, NS4/3b, NS5/1b (Kombination 4)
Sequenz 4a, 6e, 6b, 2e, 2g, NS4/3, NS5/1, 9c (Kombination 5)
Sequenz 4a, 2g, NS4/3, 2e, 6c, NS5/1, 9c (Kombination 6)
Sequenz 4a, 6, 2e, 2g, 7A1, NS5/1 (Kombination 7)

Folgende Sequenzen sind in der P 42 09 215.9 beschrieben:

| Antigen | Sequenzprotokoll in P 42 09 215.9 SEQ ID NO: | Sequenzprotokoll in der vorliegenden Anmeldung SEQ ID NO: |
|---|---|---|
| 4a | 13 | 12 |
| 6b | 18 | 13 |
| 6e | 21 | 14 |
| 2e | 7 | 15 |
| 2g | 9 | 16 |
| 6c | 19 | 17 |
| 9c | 28 | 18 |
| 6 | 16 | 19 |

In diesen Kombinationen werden die Antigene bevorzugt in folgenden Mengen verwendet:

| | Menge in Kombination [ng/ml] | |
|---|---|---|
| Antigen | Bereich | bevorzugter Bereich |
| 4a | 20 - 200 | 40 - 70 |
| 6e | 20 - 200 | 50 - 80 |
| 6b | 20 - 200 | 40 - 70 |
| 2e | 5 - 100 | 15 - 30 |
| 2g | 5 - 75 | 15 - 25 |
| NS4/3 | 10 - 120 | 25 - 40 |
| NS5/1 | 3 - 25 | 5 - 10 |
| 6c | 30 - 500 | 120 - 170 |
| NS4/3a | 20 - 200 | 45 - 60 |
| NS4/3b | 30 - 250 | 80 - 90 |
| NS5/1b | 40 - 400 | 120 - 140 |
| 9c | 100 - 1000 | 300 - 400 |
| 7A1 | 10 - 120 | 25 - 40 |
| 6 | 20 - 200 | 50 - 80 |
| 8C3 | 100 - 750 | 200 - 350 |

Vorzugsweise werden die Antigene einzeln, ohne kovalente Bindung aneinander, oder kovalent aneinander gebunden unter Verwendung eines Peptid-Linkers eingesetzt.

Auf Grund der erhöhten Empfindlichkeit, die bei dem Infektionsparameter HCV notwendig ist, werden vorzugsweise zum Nachweis heterogene Immunoassays verwendet. Diese heterogenen Tests erlauben Waschschritte, die den Meßsignal-Hintergrund erheblich reduzieren wodurch die Empfindlichkeit gesteigert wird.

Beispielsweise kann die Bestimmung durch einen Radio-Immunoassay, Enzym-Immunoassay oder durch Immunfloureszenz erfolgen. Üblicherweise wird hierzu das Peptidantigen immobilisiert. Die Probe, welche auf Anti-HCV-Antikörper untersucht werden soll, wird zugegeben und die an das Antigen gebundenen Antikörper über einen markierten Anti-Human-Immunglobulin-Antikörper bestimmt. Die Immobilisierung des erfindungsgemäßen Peptidantigens kann adsorbtiv, kovalent oder über ein biologisches Bindungspaar wie Biotin/Streptavidin, Antikörper/Antigen, oder Zucker/Lektin erfolgen. Dabei wird das Peptidantigen an diesen Partner kovalent gebunden.

Die erfindungsgemäßen Peptidantigene können für Immunoassays vorzugsweise nach dem Fachmann geläufigen Methoden, beispielsweise an Kugeln (beads), Kunststoffröhrchen oder Mikrotiterplatten (bevorzugt Polystyrol oder Copolymere von Polystyrol), immobilisiert werden. Dies erfolgt vorzugsweise dadurch, daß das Peptidantigen unspezifisch an der Oberfläche adsorbiert oder kovalent an funktionalisierte oder aktivierte Oberflächen gebunden wird. Die unspezifische Adsorption kann dadurch verbessert werden, daß man das Peptidantigen mit einem Protein zu einem Konjugat verknüpft und dieses Konjugat zur Adsorption verwendet (vgl. z.B. EP-A 0 269 092). Die Bindung kann auch über einen immobilisierten Antikörper erfolgen. Dazu sollte das Peptidantigen so verändert werden, daß das Epitop nicht durch die Bindung des Antikörpers blockiert wird z.B. durch Bildung eines Peptid-Protein-Konjugats.

Die Konjugation des Peptidantigens an den Bindungspartner erfolgt vorzugsweise über einen Spacer. Dieser Spacer enthält zweckmäßig 10 - 50, vorzugsweise 10 - 30 Atome und ist ebenfalls bevorzugt ein im wesentlichen lineares Molekül. Beispiele hierfür sind Spacer aus Alkyl-, Polyether- oder Polyamidketten. In einer besonders bevorzugten Ausführungsform ist ein Peptidantigen einer Länge von 4 - 9 Aminosäuren über einen linearen Spacer von 10 - 30 Atomen an den Träger gebunden. Falls ein Spacer aus Aminosäuren verwendet werden soll, besteht dieser zweckmäßig aus Aminosäuren, die nicht der Sequenzfolge in unmittelbarer Umgebung des Peptidantigens im HCV-Gen entsprechen.

In einer bevorzugten Ausführungsform wird das erfindungsgemäße Peptidantigen kovalent an Biotin gebunden, wobei die Immobilisierung über eine Avidin/Streptavidin-Festphase erfolgt.

Ebenso geeignet sind Bestimmungsverfahren, bei denen die Detektion nicht über einen markierten Antikörper, sondern über ein markiertes, weiteres Peptidantigen, welches eine der in SEQ ID NO 1 - 20 oder 22-28 gezeigten Sequenzen oder eine Teilsequenz davon aufweist, erfolgt.

Die Herstellung der erfindungsgemäßen Peptidantigene ist nach den dem Fachmann geläufigen Methoden zur Peptidsynthese möglich. Ein weiterer Gegenstand der Erfindung ist deshalb ein Verfahren zur Herstellung des erfindungsgemäßen Peptidantigens, welches darin besteht, daß die das C-terminale Ende bildende Aminosäure an einen Träger gebunden wird, vom C-terminalen Ende das Peptidantigen schrittweise aufgebaut und anschließend vom Träger abgespalten wird.

Im einzelnen wird dazu eine Aminosäure beispielsweise über ihre Carboxygruppe an ein unlösliches, leicht filtrierbares Polymer geknüpft, und dann vom C-terminalen Ende her die Peptidkette schrittweise aufgebaut. Zu diesem Zweck wird eine N-geschützte Aminosäure mit einer reaktiven Gruppierung des Kunstharzes zur Reaktion gebracht. Von der am Trägerpartikel kovalent verankerten Aminosäure wird die N - Schutzgruppe entfernt und das resultierende Aminoacylpolymer mit der nächsten N-geschützten Aminosäure umgesetzt. Von dem am Trägerharz kovalent gebundenen Dipeptid wird die Nα-Schutzgruppe entfernt und das resultierende Aminoacylpolymer mit der nächsten N-geschützen Aminosäure umgesetzt. Alle überschüssigen Reagentien und Beiprodukte werden durch einfaches Filtrieren entfernt. Ist die gewünschte Peptidsequenz auf diese Weise hergestellt, wird die kovalente Bindung zwischen der C-terminalen Aminosäure und der Ankergruppierung des polymeren Trägers gespalten. Der unlösliche Träger wird durch einfache Filtration von dem nun in Lösung befindlichen Peptid entfernt. Das Peptid wird mittels chromatographischer Methoden gereinigt.

Beispielsweise können die erfindungsgemäßen Peptidantigene nach Merrifield, JACS 85 (1964) 2146 hergestellt werden. Eine gegebenenfalls erforderliche Biotinylierung kann beispielsweise nach PNAS USA 80 (1983) 4045 erfolgen. Ein bevorzugtes Biotinylierungsmittel hierfür ist Biotinylaminocapronsäure-N-hydroxysuccinimidester.

Ein bevorzugtes Verfahren zur Herstellung von biotinylierten Peptidantigenen ist die Einführung des Biotinrestes am N-Terminus während einer Festphasensynthese des Peptidantigens.

Dieses Verfahren wird bevorzugt dann verwendet, wenn das Peptidantigen mehrere ε-Lysin-Aminogruppen enthält, die nicht biotinyliert werden sollen. Dies ist beispielsweise dann der Fall, wenn man N-α-Fmoc-N-ε-biotinyl-aminocaproyl)lysin, N-α-Fmoc-N-ε-Biotinyllysin oder bei Biotinylierung der N-terminalen Aminosäuren Biotin, Biotinylaminocapronsäure oder Dimethoxytritylbiotin mit einem Aktivierungsreagenz wie zum Beispiel Dicyclohexylcarbodiimid oder als Aktivester einsetzt.

In einer weiteren bevorzugten Ausführungsform wird ein Nachweisantikörper der beispielsweise gegen den Fc-Teil von humanem IgG gerichtet ist, immobilisiert. Vorzugsweise wird hierzu ein monoklonaler Antikörper verwendet. Das Peptidantigen befindet sich dann in Lösung. Der nachzuweisende Antikörper (Analyt) und auch alle anderen Antikörper der Probenflüssigkeit werden vom Wandantikörper gebunden. Der gebundene Antikörper kann dann den Analyten binden, der mit einem geeigneten Nachweissystem, z.B. kompetitiv mit einem Peptidantigen-Enzymkonjugat nachgewiesen werden kann.

Mit den erfindungsgemäßen Peptidantigenen ist es auch möglich, durch die dem Fachmann geläufigen Verfahren der Immunisierung Antikörper zu erhalten, mit denen das Virus selbst in einem immunologischen Test nachgewiesen werden kann.

Ein weiterer Gegenstand der Erfindung ist deshalb ein Verfahren zur Herstellung von Antikörpern, welches dadurch gekennzeichnet ist, daß ein Säugetier mit einem erfindungsgemäßen Peptid, welches gegebenenfalls an einen Träger gebunden ist, immunisiert wird und die Antikörper nach bekannten Verfahren z.B. aus dem Serum oder der Milz gewonnen werden.

In einer bevorzugten Ausführungsform werden B-Lymphozyten der immunisierten Tiere in Gegenwart von transformierenden Agenzien mit einer geeigneten Zellinie fusioniert, die Zellinie, welche die gewünschten Antikörper produziert, kloniert und kultiviert und aus den Zellen oder dem Kulturüberstand die monoklonalen Antikörper gewonnen.

Mit diesem Antikörper ist es möglich, HCV-Viren direkt zu bestimmen. Ein weiterer Gegenstand der Erfindung ist deshalb ein Verfahren zur Bestimmung von HCV-Viren, welches dadurch gekennzeichnet ist, daß die Probe mit einem erfindungsgemäßen Antikörper unter Bedingungen, die eine Antigen-Antikörperkomplexbildung erlauben, inkubiert, und die Menge des gebildeten Antikörper-Antigenkomplexes bestimmt wird.

Ein weiterer Gegenstand der Erfindung ist ein Verfahren zur Herstellung von Impfstoffen unter Verwendung der erfindungsgemäßen Peptidantigene sowie ein Vakzin zur Behandlung von HCV-Infektionen, enthaltend als Immunogen mindestens ein gegebenenfalls trägergebundenes Peptidantigen mit der in SEQ ID NO 1 - 11, 20 und 22 - 28 gezeigten Sequenz oder Teilsequenzen davon in einer pharmakologisch effektiven Dosis und in einer pharmazeutisch akzeptablen Formulierung.

Die Herstellung dieser Impfstoffe kann nach den bekannten Methoden durchgeführt werden. Bevorzugt werden jedoch die Peptidantigene zunächst lyophilisiert und anschließend, ggf. unter Zusatz von Hilfsstoffen, suspendiert.

Die Impfung mit dein erfindungsgemäßen Vakzin oder Vakzinkombinationen kann durch die dem Fachmann geläufigen Methoden erfolgen, beispielsweise intradermal, intramuskulär, intraperitoneal, intravenös, subkutan und intranasal.

Zur intramuskulären oder subkutanen Gabe kann das Vakzin beispielsweise in physiologischer Kochsalzlösung suspendiert sein. Zur intranasalen oder intraoccularen Applikation kann das Vakzin, beispielsweise in Form eines Sprays oder einer wäßrigen Lösung angewendet werden. Für lokale, beispielsweise orale Gabe ist es häufig erforderlich, die Immunogene zeitweise gegen Inaktivierung zu schützen, beispielsweise gegen proteolytische Enzyme in der Mundhöhle oder im Magen. Ein derartiger vorübergehender Schutz kann beispielsweise durch Verkapselung der Immunogene erfolgen. Diese Verkapselung kann beispielsweise durch Überziehen mit einem Schutzmittel (Mikroverkapselung) oder bei Einbettung einer Vielzahl von erfindungsgemäßen Immunogenen in einen schützenden Träger (Makroverkapselung) erfolgen.

Das Verkapselungsmaterial kann semipermiabel sein oder beim Einbringen in den menschlichen oder tierischen Körper semipermeabel werden. Üblicherweise wird für die Verkapselung eine biologisch abbaubare Substanz als Träger verwendet.

Ebenfalls Gegenstand der Erfindung ist ein immunologisches Verfahren zur Bestimmung infektiöser HCV-Seren mittels eines oder mehrerer Peptidantigene, wobei ein Peptidantigen aus dem carboxyl-terminalen NS4-Epitop von HCV, insbesondere außerhalb von C100-3, eingesetzt wird. Als besonders reaktiv hat sich das Peptid mit SEQ. ID. NO. 6 herausgestellt. Als infektiöse Seren werden Seren bezeichnet, in denen auch HCV RNA nachweisbar ist. Dies kann beispielsweise durch Polymerasekettenreaktion geschehen. Zu diesen Peptidantigenen gehören insbesondere solche, die eine Aminosäuresequenz enthalten, die sich um nicht mehr als eine Aminosäure von SEQ. ID. NO. 6 unterscheidet und die um nicht mehr als drei Aminosäuren gegenüber SEQ. ID. NO. 6 verkürzt oder um nicht mehr als 25 Aminosäuren verlängert oder genauso lang sind.

Die nachfolgenden Beispiele und Sequenzprotokolle erläutern die Erfindung weiter.

In den Sequenzprotokollen bedeuten:

| Antigen | SEQ ID NO: |
|---|---|
| 7B6 | 1 |
| 7A5 | 2 |
| NS5/1 | 3 |
| 7B12 | 4 |
| 6F10 | 5 |
| 7A1 | 6 |
| NS4/3a | 7 |
| NS4/3b | 8 |
| NS4/3 | 9 |
| 7D/2 | 10 |
| NS5/1b | 11 |
| 4a | 12 |
| 6b | 13 |
| 6e | 14 |
| 2e | 15 |
| 2g | 16 |
| 6c | 17 |
| 9c | 18 |
| 6 | 19 |
| 8C3 | 20 |
| B3 | 21 |
| B4 | 22 |
| B5 | 23 |
| B6 | 24 |
| D1 | 25 |
| D2 | 26 |
| D3 | 27 |
| X | 28 |

### Beispiel 1

### Synthese von H-SRRFAQALPVWARPD-OH (NS5/1)

Das Peptid wurde mittels Fmoc(Fluorenylmethoxycarbonyl)-Festphasensynthese hergestellt. Die Reaktionen wurden an einem Labortec (Schweiz) SP 640 Peptidsynthesizer durchgeführt. Die Kupplungsreaktionen wurden bezüglich Fmoc-Aminosäure-Derivat mit 2.4 Äquivalenten Dicyclohexylcarbodiimid und 2.2 Äquivalenten N-Hydroxybenzotriazol während 90 Minuten durchgeführt. Als Reaktionsmedium kam Dimethylformamid zum Einsatz. Die Fmoc-Gruppe wurde mittels 20 % Piperiden in DMF in 10 und 20 Minuten abgespalten. 2.0 Äquivalente von den folgenden Aminosäure-Derivaten kamen zum Einsatz: Pro, Arg(mit PMC (Pentamethylchroman)-Schutzgruppe), Ser(mit tert.-Butyl-Schutzgruppe), Trp, Asp(mit tert.-Butylester-Schutzgruppe), Phe, Ala, Gln, Leu, Val. Die Kupplungsreaktionen wurden mit der Hälfte der Reagentien wiederholt. Der Kupplungserfolg wurde mittels Kaiser-Test (Anal. Biochemistry 34(1970)595) geprüft, die Harzbeladung wurde mittels UV-Absorption der freigesetzten Fulven-Gruppe nach jeder Piperidin-Spaltung ermittelt. Das Peptid wurde an 5 g Wang-Harz (Polystyrol/1 % Divinylbenzol) mit einer Ladung von 0.50 mMol/g synthetisiert (JACS 95(1973)1328). Nach der Synthese betrug der Beladungsgrad noch 0.39 mMol/g.

Die Freisetzung des Peptids wurde mit 200 ml Trifluoressigsäure, 200 ml Dichlormethan, 10 ml Ethandithiol, 10 ml m-Kresol, 5 ml Ethylmethylsulfid und 5 ml Wasser in 30 Minuten bei Raumtemperatur. Die Abspaltlösung wurde mehrmals mit Toluol eingeengt, dann das Peptid mit Diethylether gefällt.

Zur Entfernung der Scavenger und anderer kleiner Moleküle wurde das Rohmaterial über eine Sephadex G10-Säule gereinigt. Es wurden nach Lyophilisieren 2.4 g Material einer Reinheit von 31 % (RP-HPLC) erhalten. Um das Material auf die Endreinheit von > 95 % zu bringen, wurden 250 mg Peptid über eine präparative RP-HPLC-Säule (40mmx250mm) gefüllt mit C18-Material (5 Mikrometer, 300 Angström) und einem Wasser/Trifluor-essigsäure-, Acetonitril/Trifluoressigsäure-Gradienten gereinigt. Es fielen nach Lyophilisation 48 mg 95.2 %iges (HPLC) weißes Material an. Die Identität des Materials wurde mittels FAB-MS geprüft.

### Beispiel 2

Zur Biotinylierung des Peptidantigens aus Beispiel 1 wurde ein Moläquivalent möglichst konzentriert (Löslichkeit ist von der Aminosäuresequenz abhängig) in Argon-gesättigtem Kaliumphosphatpuffer (0,1 mol/l, pH 8,0) gelöst und mit 3 Äquivalenten D-Biotinyl-ε-aminocapronsäure-N-hydroxysuccinimidester gelöst in Argon-gesättigtem Dimethylformamid (Lösung von 1 µmol Reagenz in 5 µl DMF) versetzt.

Man rührte das Reaktionsgemisch 2 Stunden bei Raumtemperatur unter Argon unter ständiger Kontrolle mittels analytischer RP-HPLC. Wenn < 5% Edukt vorhanden waren, wurde der Reaktionsansatz direkt auf eine präparative RP-HPLC-Säule gegeben und das Produktmaterial mittels einem 0.1% Trifluoressigsäure/Wasser- zu 0.1% Trifluoressigsäure/Acetonitril-Gradienten (Steigung: 0% auf 100% in 90 Minuten) gereinigt. Das Produktmaterial wurde durch Einengen und Lyophilisation der Produktfraktionen erhalten. Die Ausbeuten lagen zwischen 40 % und 90%. Als Analytik dienten für die Reinheit HPLC, HPCE und TLC, für die Identität LSI-MS (Molpeak) und TLC mit spezifischen Anfärbereagentien (p-Dimethylaminozimtaldehyd auf Biotin) und Gehaltsbestimmung über Mikroanalyse (Nitrogen).

### Beispiel 3

HCV-Antikörper werden in einem 2-Schritt-Sandwich-Immunoassay bestimmt. Zum Nachweis werden die Reagentien mit folgender Zusammensetzung verwendet :

### Reagenz 1:

Kombinationen 1 - 6 der Peptidantigene, biotinylierte Peptidantigene oder Peptidantigene allein.
40 mmol/1 Phosphatpuffer pH 7.0
0.9 Gew.-% NaCl
10 Vol.-% Rinderserum

### Reagenz 2:

20 mU/ml eines Konjugats aus polyklonalem Antikörper gegen humanes Immunglobulin (Schaf) und Peroxidase
40 mmol/l Phosphatpuffer pH 7.0
0.05 Gew.-% Tween^{R} 20

0.2% Rinderserumalbumin
0.2% Rinder-IgG

In einem mit Streptavidin beschichteten Polystyrolröhrchen (hergestellt nach Beispiel 1 von EP-A 0 344 578) werden 1 ml Reagenz 1 und 10 µl Probe eine Stunde bei Raumtemperatur inkubiert. Anschließend wird dreimal mit Leitungswasser gewaschen und mit 1 ml Reagenz 2 für eine Stunde bei Raumtemperatur inkubiert. Anschließend wird dreimal mit Leitungswasser gewaschen. Zur Nachweisreaktion werden 1 ml ABTS^{c} (2,2' -Azino-di-[3-ethyl-benzthiazolinsulfonat (6) ]-Diammoniumsalz, 1,9 mmol/l, in 100 mmol/l Phosphatcitratpuffer pH 4.4 mit 3.2 mmol/l Natrium-perborat) zugegeben. Nach 60 min wird die Extinktion bei 420 nm photometrisch gemessen. Die Ergebnisse zeigen die Tabellen I, II und III.

Erläuterungen zu den Tabellen:

-/+: negativ/positiv (Der Cutoff für ein positives Signal im ELISA ist definiert als die mittlere Extinktion bei 420 nm plus 3 Standardabweichungen eines Kollektivs von 10 negativen Kontrollseren. Die Proben wurden bei einer Probenverdünnung von 1:250 vermessen).

Als Antigenkonzentrationen wurden verwendet:
a) als einzelnes Antigen im Test

| Antigen | Menge [ng/ml] |
|---|---|
| 7B6 | 200 |
| 7A5 | 200 |
| NS5/16 | 130 |
| NS5/1 | 85 |
| 7D2 | 200 |
| 7B12 | 200 |
| 6F10 | 70 |
| 7A1 | 200 |
| 8C3 | 300 |

b) in den Kombinationen

| | Menge [ng/ml] in Kombination | | | | | | |
|---|---|---|---|---|---|---|---|
| Antigen | 1 | 2 | 3 | 4 | 5 | 6 | 7 |
| 4a | 52 | 65 | 52 | 50 | 50 | 52 | 52 |
| 6e | 58 | 73 | - | 58 | 55 | - | - |
| 6b | 52 | 65 | - | 50 | 50 | - | - |
| 2e | 20 | 25 | 20 | 20 | 18 | 20 | 20 |
| 2g | 17 | 21 | 17 | 17 | 15 | 17 | 17 |
| NS4/3 | 30 | 38 | 30 | - | 27 | 30 | - |
| NS5/1 | 7 | - | 7 | - | 7 | 7 | 7 |
| 6c | - | - | 150 | - | - | 150 | - |
| NS4/3a | - | - | - | 51 | - | - | - |
| NS4/3b | - | - | - | 83 | - | - | - |
| NS5/1b | - | - | - | 130 | - | - | - |
| 9c | - | - | - | - | 350 | 350 | - |
| 7A1 | - | - | - | - | - | - | 30 |
| 6 | - | - | - | - | - | - | 65 |

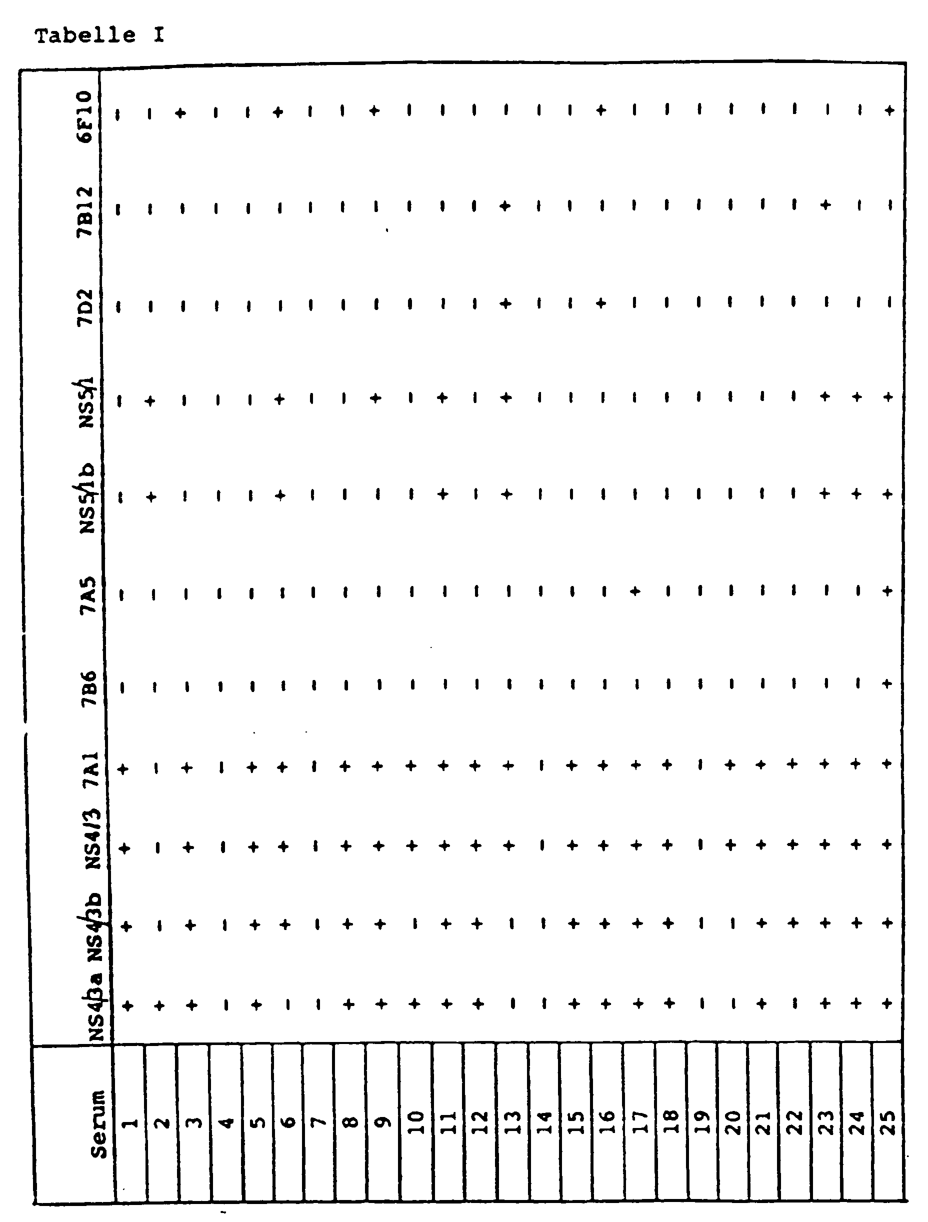

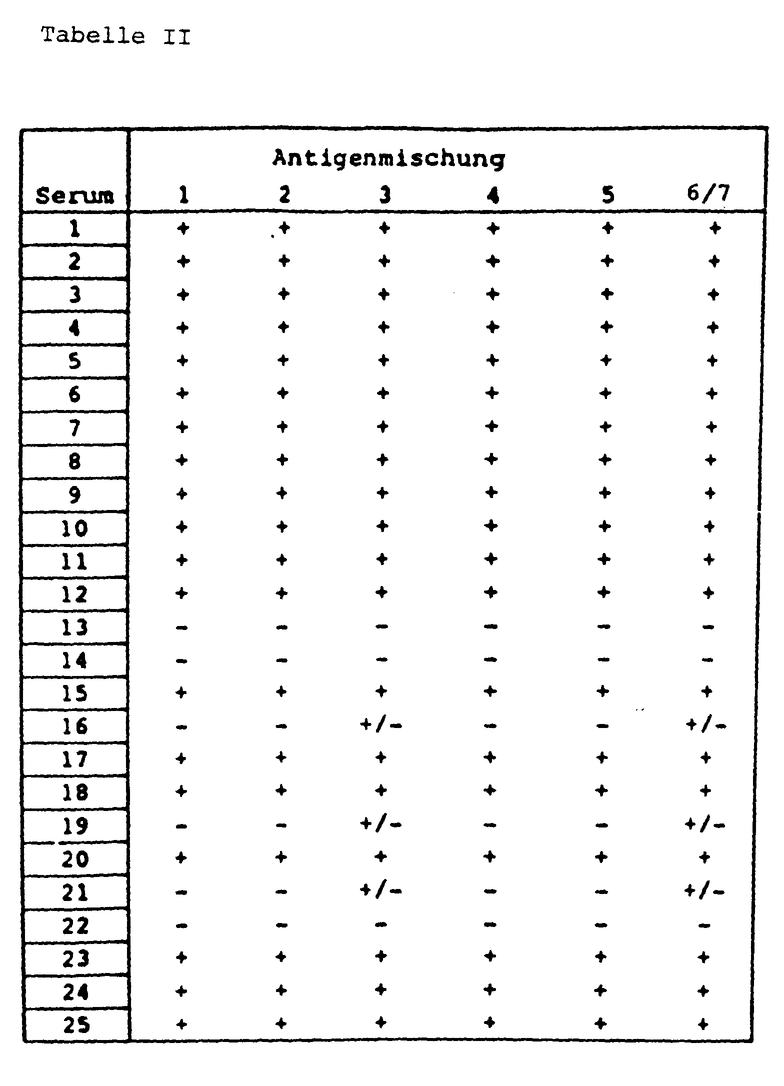

**Tabelle III**

| Serum | Antigen 8C3 |
|---|---|
| B1 | - |
| B2 | - |
| B3 | + |
| B4 | +/- |
| B5 | - |
| B6 | - |
| B7 | +/- |
| B8 | + |
| B9 | + |
| B10 | + |
| B11 | - |
| B12 | - |
| B13 | + |
| B14 | + |
| B15 | - |
| B16 | - |
| B17 | + |
| B18 | + |
| B19 | + |
| B20 | + |
| S25 | + |
| 01 | - |
| 56 | + |
| Negativ-control | - |

### Beispiel 4

In einer Studie mit klinischen Proben wurden 9 infektiöse Seren mit PCR aufgefunden und mittels Immunoassay weiter untersucht. Davon wurden 2 mit einer herkömmlichen Antigenmischung (c22-3, c33c, c100-3 ) erkannt. Das Peptid 7A1 führt zu der Erkennung von einem zusätzlichen Serum.

**Tabelle IV**

| Probennr. | PCR | HCV-Antigene (c22-3, c33c, c100-3) | 7A1 |
|---|---|---|---|
| 1 | +/+ | - | - |
| 2 | +/+ | - | + |
| 3 | +/+ | +++ | +++ |
| 4 | +/+ | - | - |
| 5 | +/+ | ++ | +++ |
| 6 | +/+ | - | - |
| 7 | +/+ | - | - |
| 8 | +/+ | - | - |
| 9 | +/+ | - | - |

### Beispiel 5

Unter 2000 Blutspendern wurden mit dem Peptid 7A1 5 infektiöse Seren detektiert. Davon werden nur 2 mit einer herkömmlichen Antigenmischung (c22-3, c33c, c100-3) erkannt

**Tabelle V**

| Probennr. | PCR | HCV-Antigene (c22-3 , c33c, c100-3) | 7A1 |
|---|---|---|---|
| 1 | +/+ | +++ | ++ |
| 2 | +/+ | +++ | +++ |
| 3 | +/- | - | + |
| 4 | +/- | - | +/- |
| 5 | +/- | - | ++ |

### Beispiel 6

### Epitop in der core-Region

Im Folgenden ist ein neues dominantes HCV-Antigen in der core-Region beschrieben. Dieses Antigen konnte mittels klassischer Methoden nicht gefunden werden, sondern nur unter Verwendung biotinylierter Peptide analog zu Beispiel 2 und Austesten der Fähigkeit der einzelnen biotinylierten Peptide, mit Antikörpern aus Seren, die nachgewiesenerweise HCV-positiv sind, Immunkomplexe zu bilden. Diese Peptide sind für alle Verfahren bei denen lösliche Peptidantigene verwendet werden besonders brauchbar, weniger jedoch in Verfahren bei denen direkt an eine Festphase gebundene Peptidantigene verwendet werden.

Die längste Sequenz ist Antigen X (SEQ. ID. NO. 28). Die kürzeste und reaktivste Sequenz ist D2 (SEQ. ID. NO. 26). Das reaktivste Antigen (höchstes Signal im Immunoassay nach Beispiel 3) ist D1 (SEQ. ID. NO. 25). Eine Restreaktivität zeigt auch D3 (SEQ. ID. NO. 27). Die Sequenzen wurden mit 26 HCV positiven Seren getestet und zeigten mit allen eine deutliche Reaktion (24 Stück > 500 mE, eines > 300 mE und eines > 150 mE), jedoch keine Reaktion mit Negativserum (70 mE). Ferner wurden die Antigene B3 (SEQ. ID. NO. 21), B4 (SEQ. ID. NO. 22), B5 (SEQ. ID. NO. 23) und B6 (SEQ. ID. NO. 24) getestet und deren Signalhöhenabhängigkeit von der Antigenkonzentration gemessen. Daraus ergeben sich typische Einsatzkonzentrationen der Antigene B4 bis B6 von 150 - 200 µm/ml in Tests analog zu Beispiel 3. B3 ist nicht reaktiv.

Die Ergebnisse der Austestung der genannten neuen Peptide ist in den Tabellen VI und VII enthalten. Es wurden jeweils 50 ng des Antigens in 100 µl Inkubationspuffer verwendet. Die Seren wurden 1:60 verdünnt. Die Reaktivitäten bedeuten:
- + :: 70-140 mE
- ++:: 40-500 mE
- +++:: > 500 mE

**TABELLE VI**

| Serum | Antigene | | | |
|---|---|---|---|---|
| | B 4 | B 5 | B 6 | Referenzpeptid |
| neg | --- | --- | --- | --- |
| 071 | +++ | ++ | +++ | + |
| 075 | n. b. | n.b | n.b. | n.b. |
| 575 | +++ | +++ | +++ | --- |
| 004 | +++ | +++ | +++ | --- |
| 069 | +++ | +++ | ++ | --- |
| 56- | +++ | +++ | +++ | --- |
| B 1 | +++ | ++ | ++ | --- |
| B 2 | +++ | ++ | +++ | --- |
| B 3 | +++ | ++ | ++ | ++ |
| B 4 | +++ | ++ | ++ | --- |
| B 5 | +++ | +++ | ++ | --- |
| B 6 | ++ | ++ | ++ | --- |
| B 7 | ++ | ++ | ++ | --- |
| B 8 | ++ | +++ | +++ | --- |
| B 9 | ++ | +++ | ++ | --- |
| B 10 | +++ | +++ | +++ | + |
| B 11 | ++ | ++ | ++ | --- |
| B 12 | n.b. | n.b. | n.b. | n.b. |
| B 13 | ++ | +++ | +++ | --- |
| B 14 | ++ | +++ | +++ | --- |
| B 15 | ++ | ++ | ++ | --- |
| B 16 | ++ | ++ | ++ | + |
| B 17 | ++ | +++ | +++ | --- |
| B 18 | ++ | +++ | +++ | --- |
| B 19 | ++ | +++ | +++ | ++ |
| B 20 | ++ | + | + | --- |

**TABELLE VII**

| Serum | Antigene | | | |
|---|---|---|---|---|
| | D 1 GVYLLPRR | D 2 VYLLPR | D 3 YLLP | Referenzpeptid |
| neg | --- | --- | --- | --- |
| 071 | +++ | ++ | + | --- |
| 075 | +++ | ++ | ++ | --- |
| 575 | +++ | ++ | + | --- |
| 004 | +++ | ++ | ++ | --- |
| 069 | +++ | + | --- | --- |
| 56- | +++ | ++ | --- | --- |
| B 1 | +++ | ++ | ++ | --- |
| B 2 | +++ | ++ | ++ | --- |
| B 3 | +++ | ++ | ++ | --- |
| B 4 | +++ | +++ | + | --- |
| B 5 | +++ | +++ | ++ | --- |
| B 6 | +++ | ++ | --- | --- |
| B 7 | +++ | ++ | ++ | --- |
| B 8 | +++ | ++ | +/- | --- |
| B 9 | +++ | +++ | +++ | --- |
| B 10 | +++ | +++ | +++ | + |
| B 11 | +++ | ++ | + | + |
| B 12 | + | + | --- | --- |
| B 13 | +++ | +++ | --- | --- |
| B 14 | +++ | +++ | +++ | --- |
| B 15 | ++ | ++ | --- | --- |
| B 16 | +++ | ++ | --- | --- |
| B 17 | +++ | +++ | ++ | --- |
| B 18 | +++ | ++ | +/- | --- |
| B 19 | +++ | +++ | + | --- |
| B 20 | ++ | ++ | + | --- |

## Patentansprüche

1. HCV Peptidantigene mit der Aminosäuresequenz SEQ ID NO: 28 oder mit Teilsequenzen davon mit mindestens 4 Aminosäuren Länge.

2. HCV Peptidantigene nach Anspruch 1 mit Teilsequenzen von maximal 9 Aminosäuren Länge.

3. HCV Peptidantigene mit den Aminosäuresequenzen SEQ ID NO: 22 - 27.

4. HCV Peptidantigen, welches SEQ ID NO: 26 enthält.

5. Verfahren zur Herstellung von Antikörpern gegen HCV-Antigene, dadurch gekennzeichnet, daß ein Säugetier mit einem gegebenenfalls trägergebundenen Peptidantigen SEQ ID NO: 28 oder einer Teilsequenz davon immunisiert wird, polyklonale Antikörper gewonnen werden oder Zellen dieser Tiere, die Antikörper produzieren, zu Zellinien immortalisiert werden und aus diesen Zellinien monoklonale Antikörper gewonnen werden.

6. Verfahren nach Anspruch 5, dadurch gekennzeichnet, daß als Peptidantigene, die Teilsequenzen darstellen, SEQ ID NO: 22 - 27 verwendet werden.

7. Verfahren zur Bestimmung von HCV-Viren, dadurch gekennzeichnet, daß die Probe mit einem Antikörper nach Anspruch 5 oder 6 unter Bedingungen, die eine Antigen-Antikörperkomplexbildung erlauben, inkubiert wird, und die Menge des gebildeten Antikörper-Antigenkomplexes bestimmt wird.

8. Vakzin zur Behandlung von HCV-Infektionen, enthaltend mindestens ein gegebenenfalls trägergebundenes Peptidantigen SEQ ID NO: 28 oder Peptidantigene, die Teilsequenzen dieses Peptidantigenes von mindestens 4 Aminosäuren Länge darstellen als Immunogen, in einer pharmakologisch effektiven Dosis und in einer pharmazeutisch akzeptablen Formulierung.

9. Verfahren zur Herstellung von Impfstoffen unter Verwendung des Peptidantigenes SEQ ID NO: 28 oder von Peptidantigenen, die Teilsequenzen dieser Peptidantigene von mindestens 4 Aminosäuren Länge darstellen, als Immunogene.

10. Immunologisches Verfahren zur Bestimmung infektiöser HCV-Seren, mittels eines oder mehrerer Peptidantigene, dadurch gekennzeichnet, daß ein Peptid gemäß Anspruch 1 bis 4 aus dem core-Epitop von HCV eingesetzt wird.

11. Antigene nach Anspruch 1 bis 4, dadurch gekennzeichnet, daß an die Aminosäuresequenz nicht HCV-spezifische Reste, vorzugsweise Biotinreste oder/und nicht HCV-spezifische Aminosäuresequenzen gebunden sind.


SEQUENZPROTOKOLL

(1) ALLGEMEINE INFORMATION:

(i) ANMELDER:
(A) NAME: Boehringer Mannheim GmbH
(B) STRASSE: Sandhofer Strasse 116
(C) ORT: Mannheim
(E) LAND: DE
(F) POSTLEITZAHL: 68298
(G) TELEPHON: 0621 759 4348
(H) TELEFAX: 0621 759 4457

(ii) ANMELDETITEL: HCV Peptidantigene und Verfahren zur
Bestimmung von HCV

(iii) ANZAHL DER SEQUENZEN: 28

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 18 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 1:

Leu Asp Gly Val Arg Leu His Arg Phe Ala Pro Pro Cys Lys Pro Leu
1               5                   10                  15

Leu Arg

(2) INFORMATION ZU SEQ ID NO: 2:

(i) SEQUENZ CHARAKTERISTIKA:
(A) LÄNGE: 21 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 2:

Leu His Gln Trp Ile Ser Ser Glu Cys Thr Thr Pro Cys Ser Gly Ser
1               5                   10                  15

Trp Leu Arg Asp Ile
            20

(2) INFORMATION ZU SEQ ID NO: 3:

(i) SEQUENZ CHARAKTERISTIKA:
(A) LÄNGE: 15 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 3:

Ser Arg Arg Phe Ala Gln Ala Leu Pro Val Trp Ala Arg Pro Asp
1               5                   10                  15

(2) INFORMATION ZU SEQ ID NO: 4:

(i) SEQUENZ CHARAKTERISTIKA:
(A) LÄNGE: 21 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 4:

Asn Lys Val Val Ile Leu Gly Ser Phe Asp Pro Leu Val Ala Glu Glu
1               5                   10                  15

Asp Glu Arg Glu Ile
            20

(2) INFORMATION ZU SEQ ID NO: 5:

(i) SEQUENZ CHARAKTERISTIKA:
(A) LÄNGE: 16 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 5:

Pro Ser His Ile Thr Ala Glu Ala Ala Gly Arg Arg Leu Ala Arg Gly
1               5                   10                  15

(2) INFORMATION ZU SEQ ID NO: 6:

(i) SEQUENZ CHARAKTERISTIKA:
(A) LÄNGE: 18 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 6:

Ser Arg Gly Asn His Val Ser Pro Thr His Tyr Val Pro Glu Ser Asp
1               5                   10                  15

Ala Ala

(2) INFORMATION ZU SEQ ID NO: 7:

(i) SEQUENZ CHARAKTERISTIKA:
(A) LÄNGE: 9 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 7:

His Val Ser Pro Thr His Tyr Val Pro
1               5

(2) INFORMATION ZU SEQ ID NO: 8:

(i) SEQUENZ CHARAKTERISTIKA:
(A) LÄNGE: 9 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 8:

Val Ser Pro Thr His Tyr Val Pro Glu
1               5

(2) INFORMATION ZU SEQ ID NO: 9:

(i) SEQUENZ CHARAKTERISTIKA:
(A) LÄNGE: 10 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 9:

His Val Ser Pro Thr His Tyr Val Pro Glu
1               5                   10

(2) INFORMATION ZU SEQ ID NO: 10:

(i) SEQUENZ CHARAKTERISTIKA:
(A) LÄNGE: 9 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 10:

Ala Leu Pro Val Trp Ala Arg Pro Asp
1               5

(2) INFORMATION ZU SEQ ID NO: 11:

(i) SEQUENZ CHARAKTERISTIKA:
(A) LÄNGE: 9 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 11:

Phe Ala Gln Ala Leu Pro Val Trp Ala
1               5

(2) INFORMATION ZU SEQ ID NO: 12:

(i) SEQUENZ CHARAKTERISTIKA:
(A) LÄNGE: 9 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 12:

Lys Asn Lys Arg Asn Thr Asn Arg Arg
1               5

(2) INFORMATION ZU SEQ ID NO: 13:

(i) SEQUENZ CHARAKTERISTIKA:
(A) LÄNGE: 9 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 13:

Lys Phe Pro Gly Gly Gly Gln Ile Phe
1               5

(2) INFORMATION ZU SEQ ID NO: 14:

(i) SEQUENZ CHARAKTERISTIKA:
(A) LÄNGE: 9 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 14:

Gln Asp Val Lys Phe Pro Gly Gly Gly
1               5

(2) INFORMATION ZU SEQ ID NO: 15:

(i) SEQUENZ CHARAKTERISTIKA:
(A) LÄNGE: 9 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 15:

Ser Gln His Leu Pro Tyr Ile Glu Gln
1               5

(2) INFORMATION ZU SEQ ID NO: 16:

(i) SEQUENZ CHARAKTERISTIKA:
(A) LÄNGE: 9 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 16:

Gln Lys Ala Leu Gly Leu Leu Gln Thr
1               5

(2) INFORMATION ZU SEQ ID NO: 17:

(i) SEQUENZ CHARAKTERISTIKA:
(A) LÄNGE: 12 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 17:

Gln Asp Val Lys Phe Pro Gly Gly Gly Gln Ile Val
1               5                   10

(2) INFORMATION ZU SEQ ID NO: 18:

(i) SEQUENZ CHARAKTERISTIKA:
(A) LÄNGE: 15 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 18:

Lys Ala Arg Arg Pro Glu Gly Arg Thr Trp Ala Gln Pro Gly Tyr
1               5                   10                  15

(2) INFORMATION ZU SEQ ID NO: 19:

(i) SEQUENZ CHARAKTERISTIKA:
(A) LÄNGE: 16 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 19:

Pro Gln Asp Val Lys Phe Pro Gly Gly Gly Gln Ile Val Gly Gly Val
1               5                   10                  15

(2) INFORMATION ZU SEQ ID NO: 20:

(i) SEQUENZ CHARAKTERISTIKA:
(A) LÄNGE: 15 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 20:

Leu Leu Leu Leu Ala Ala Gly Val Gly Ile Tyr Leu Leu Pro Asn
1               5                   10                  15

(2) INFORMATION ZU SEQ ID NO: 21:

(i) SEQUENZ CHARAKTERISTIKA:
(A) LÄNGE: 11 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 21:

Pro Gly Gly Gln Ile Val Gly Gly Val Tyr Leu
1               5                   10

(2) INFORMATION ZU SEQ ID NO: 22:

(i) SEQUENZ CHARAKTERISTIKA:
(A) LÄNGE: 12 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 22:

Gly Gln Ile Val Gly Gly Val Tyr Leu Leu Pro Arg
1               5                   10

(2) INFORMATION ZU SEQ ID NO: 23:

(i) SEQUENZ CHARAKTERISTIKA:
(A) LÄNGE: 12 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 23:

Val Gly Gly Val Tyr Leu Leu Pro Arg Arg Gly Pro
1               5                   10

(2) INFORMATION ZU SEQ ID NO: 24:

(i) SEQUENZ CHARAKTERISTIKA:
(A) LÄNGE: 12 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 24:
Val Tyr Leu Leu Pro Arg Arg Gly Pro Arg Leu Gly
1               5                   10

(2) INFORMATION ZU SEQ ID NO: 25:

(i) SEQUENZ CHARAKTERISTIKA:
(A) LÄNGE: 8 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 25:

Gly Val Tyr Leu Leu Pro Arg Arg
1               5

(2) INFORMATION ZU SEQ ID NO: 26:

(i) SEQUENZ CHARAKTERISTIKA:
(A) LÄNGE: 6 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 26:

Val Tyr Leu Leu Pro Arg
1               5

(2) INFORMATION ZU SEQ ID NO: 27:

(i) SEQUENZ CHARAKTERISTIKA:
(A) LÄNGE: 4 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 27:

Tyr Leu Leu Pro
1

(2) INFORMATION ZU SEQ ID NO: 28:

(i) SEQUENZ CHARAKTERISTIKA:
(A) LÄNGE: 17 Aminosäuren
(B) ART: Aminosäure
(C) STRANGFORM: Einzel
(D) TOPOLOGIE: linear
(ii) ART DES MOLEKÜLS: cDNS

(xi) SEQUENZBESCHREIBUNG: SEQ ID NO: 28:

Gly Gln Ile Val Gly Gly Val Tyr Leu Leu Pro Arg Gly Pro Arg Leu
1               5                   10                  15

Gly